# EUROPEAN PATENT SPECIFICATION

(11) **EP 0 871 361 B1**
(45) Date of publication and mention of the grant of the patent: **01.10.2003**
(21) Application number: 96918933.1
(22) Date of filing: 10.06.1996
(51) Int. Cl.: A01N 47/34, A61K 31/17

(54) **AGENT FOR COMBATING PARASITES IN FARMED FISH**
MITTEL ZUR BEKÄMPFUNG VON PARASITEN IN FARMFISCHEN.
AGENT POUR COMBATTRE LES PARASITES EN PISCICULTURE

(30) Priority: 13.06.1995 NO 952317
(43) Date of publication of application: 21.10.1998
(73) Proprietor: NUTRECO AQUACULTURE RESEARCH CENTRE A/S, 4033 Forus (NO)
(72) Inventor: HOFF, Kjell, Arne, N-4300 Sandnes (NO); RITCHIE, Gordon, N-4050 Sola (NO)
(74) Representative: Lawrence, Malcolm Graham
(86) International application number: NO9600139
(87) International publication number: WO96041536

(56) References cited:
- WO-A-92/16106
- WO-A-96/10915
- WO-A-96/25852
- GB-A- 2 260 703
- ACTA VET. SCAND., Volume 32, No. 4, 1991, TOR EINAR HORSBERG et al., "Tissue Distribution of C-Diflubenzuron in Atlantic Salmon (Salmo Salar)", pages 527-533.
- STN INTERNATIONAL, File CABA, CABA Accession No. 93:38822, CHUI V.W.D. et al., "Laboratory Evaluation of Vectobac-12AS and Teflubenzuron Against Culex and Aedes Mosquito Larvae under Different Physical Conditions"; & ENVIRONMENT INTERNATIONAL, (1993), Vol. 19, No. 2, pp. 193-202, 30 ref.
- Ed: CHARLES R. WORTHING et al., "The Pesticide Manual, A World Compendium", Ninth Edition, 1991, THE BRITISH CROP PROTECTION COUNCIL, page 790, No. 11235, pages 281-282, No. 4720.
- BAKKE AND HARRIS: 'Diseases and parasites in wild Atlantic salmon (Salmo salar ) populations' CAN. J. FISH. AQUAT. SCI. vol. 55, 1998, CANADA, pages 247 - 266

## Description

The present invention refers to a composition for the oral treatment of farmed fish against parasite infection. The invention also refers to the use of an agent for combating parasites for the manufacture of a medicament for the treatment of farmed fish.

Fishfarming can suffer great economic loss due to external and internal parasites. For example, external crustacean parasites such as *Lepeophtheirus* and *Caligus* (sea lice) will cause huge wounds to the fish reducing the slaughter value. If the infections are not treated, the fish might as worst case die from the wounds. The fish farmer will also suffer an economic loss because of reduced growth as a result of reduced well-being of the fish.

Until now various means of bath treatment have been used in an attempt to combat these parasites. For example, gill parasites have been treated with formalin dissolved in the water in which the fish is swimming. Sea lice are treated with different kinds of organophosphates such as metriphonate, dichlorvos, and azamethiphos. Hydrogen peroxide is also used.

Pyrethrum and synthetic pyrethroides are used as an oil emulsion formulation through which the fish is forced to swim, in order to get a treatment effect.

The insecticide Invermectin is used as an oral agent against sea lice. Invermectin is mixed in the feed and this medicated feed is given to the salmon two days a week throughout the season against sea lice attacks. A laboratory trial has been reported where metriphonate has been used as an oral treatment against sea lice attacks. There is also a publication describing the use of the benzoyl-urea compound diflubenzuron against sea lice (T.E. Horseberg and T. Høy, Acta vet. scand. 1991, 32, 527-533), whilst another describes the inhibiting effect of another benzoyl-urea compound teflubenzuron on the normal development of mosquito larvae (V.W.D. Chui et al., Environment International 1993; 19:193-202). W0-A-96/25852 is a non-prepublished patent application of an earlier date, that discloses a composition for controlling parasites which comprises as active ingredient, a combination of at least one compound selected from the N-phenyl-benzoyl-urea class of substances and at least one compound selected from the milbemycin, avermectin, milbemycin-oxime, moxidectin, ivermectin, abamectin and doramectin classes of substances. It also discloses a method of controlling parasites and a process for preparation of the composition and the use thereof. The application does not disclose the use of the composition in treating fish. The application describes use of the composition against parasites of the order Acarina. The application does not mention crustacean parasites. The application discloses use of lufenuron and fluazuron as benzoyl-urea compounds.

Different kinds of wrasse (fam. Labridae), especially goldsinny, rock cook, corkwing, are used as biological controls. When these are stocked together with salmon in the proportion 1:50, the wrasse will eat sea lice from the skin of the salmon.

The substances for bath treatment (organophosphates, hydrogen peroxide) against sea lice have to be mixed into the water in which the fish is swimming. In order to prevent the water volume becoming too big, the bottom of the net is manually lifted before treatment and a tarpaulin is put around the net like a skirt, preventing dissolution and the drifting of the chemical away before the treatment has become effective. These work operations are demanding and also stressful to the fish, which shows signs of reduced appetite after treatment.

Organophosphate and hydrogen peroxide are most efficacious against the three final stages of the life cycle, out of the total 8 stages which the sea lice are going through on the fish skin. Therefore it does not take much time before new stages, causing wounds, appears on the fish, and the treatment has to be repeated.

An increasing resistance problem has been reported from Scotland and some fish farms can no longer use organophosphates as these have no efficacy any more.

The organophosphate are toxic for humans and have to be handled with care. Hydrogen peroxide is corrosive. These mixtures create a disadvantageous strain on the working environment.

correct dosage of organophosphates and hydrogen peroxide is important because the therapeutic margin is small. There are no good, rapid methods for measuring the concentration of organophosphates in water.

Pyrethrum and pyrethroides are toxic to fish when dissolved in water. In the oil emulsion the toxic effect is decreased and the oil emulsion can be coated to the fish's skin. The coating can be done either by spraying the oil emulsion over the fish at sorting, or the fish are forced to swim/glide through a pipe which is filled with oil suspension/pyrethrum in connection with sorting or moving of fish. Both methods require the fish to be taken out of the water. This is stressful for the fish and is work demanding.

Ivermectin is a broad spectered insecticide. It is slowly metabolised in fish and therefore causes a long retention time which is the time between the last treatment and the first day the level of medicine or its metabolites in the fish is so low that the slaughter can be approved for human consumption. The broad spectered effect of Ivermectin is assumed to be disadvantageous for the animal community living on the sea bottom near a fish farm.

Wrasse has shown little ability to survive throughout the winter, when not given the opportunity to seek for deeper water. This is prevented by the cages that are used in fish farming. Wrasse cannot be used for delousing of big fish, as it will be eaten by the salmon. Wrasse is not always widespread where the farms are situated. For example it is not likely to be found in Norway from Nordland and further north. Supplies of wrasse are based on natural harvests.

The purpose of the invention is to bring forward a composition for combating fish parasites, especially sea lice, which does not have the disadvantages of present treatments. This can be achieved by the invention, which provides a composition for the oral treatment of farmed fish against chitin synthesis-dependent parasite infection consisting, of teflubenzuron of the formula: 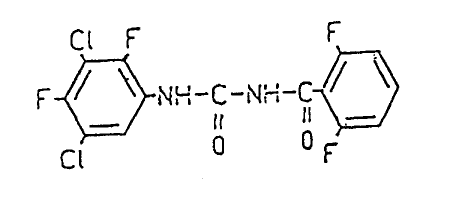 as agent for combating parasites and an edible carrier.

The active substance teflubenzuron is added to known feed substances for farmed fish.

The edible carrier may be fish food, preferably in the form of pellets.

The present invention also provides the use of teflubenzuron of the above formula for the manufacture of a medicament for the treatment of farmed fish against chitin synthesis-dependent parasite infection.

The medicament may be for oral administration, preferably wherein the medicament is in the form of fish food, more preferably in the form of pellets.

The fish to be treated may be salmon.

The parasites may be crustaceans, tapeworm, sea lice, of the family *Copopeda,* or of the species *Lepeophtheirus* or *Caligus.*

Teflubenzuron is added to produced fish feed/pellets or is mixed together with the other ingredients in the fish feed before the pellet is formed. Telflubenzuron can also be added in capsules or other means of carriers that the fish will eat.

Fish that is fed with pellets or other carriers of teflubenzuron, will take up the active form of teflubenzuron from the stomach and gut. The active form of teflubenzuron will be distributed to all parts of the body including the mucus on gills and skin. Parasites in the gut, influenced by teflubenzuron, will take in teflubenzuron together with other gut contents.

The reason why teflubenzuron is active in combating parasites such as crustaceous parasites is that the synthesis of chitin is prevented. Parasites that are dependent on chitin synthesis will die during a certain amount of time after exposure through intake of teflubenzuron. For example, crustaceans will not moult and consequently die. For sea lice in particular the teflubenzuron will work on all stages which moult while the parasites is attached to or is moving on the fish skin. In addition, teflubenzuron will affect the quality of the eggs produced by females after treatment. The eggs will either not be non-viable or they will hatch in an abnormal way or the released naupli will be deformed or not able to go through a normal life cycle.

In order to prove the effect of teflubenzuron as an agent against sea lice, the following trials were done:

### Trial 1

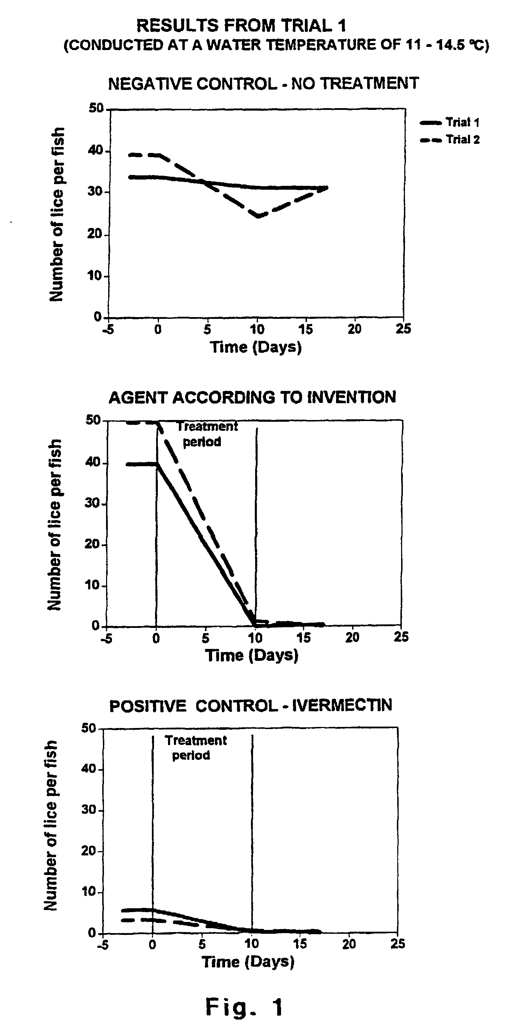

Salmon (salmo salar) kept in tanks were artificially infected by adding a great number of infectious larvae to the water. Seven days after infection the fish were treated by adding to the salmon a daily dose of 200 mg teflubenzuron per kilo body weight. The period of treatment was 10 days. Teflubenzuron was administered through the feed. Teflubenzuron and ordinary fish feed were mixed such that teflubenzuron was spread on the surface of feed pellets. The fish ate the feed voluntarily. The concentration of teflubenzuron in the feed was 20 g/kg feed. The temperature of the water was 11-14.5 °C. The result is compared with a group of fish that was not treated and a group of fish that was treated with Ivermectin two days a week before infection and after infection. Figure 1 shows that teflubenzuron is as effective as Ivermectin.

### Trial 2

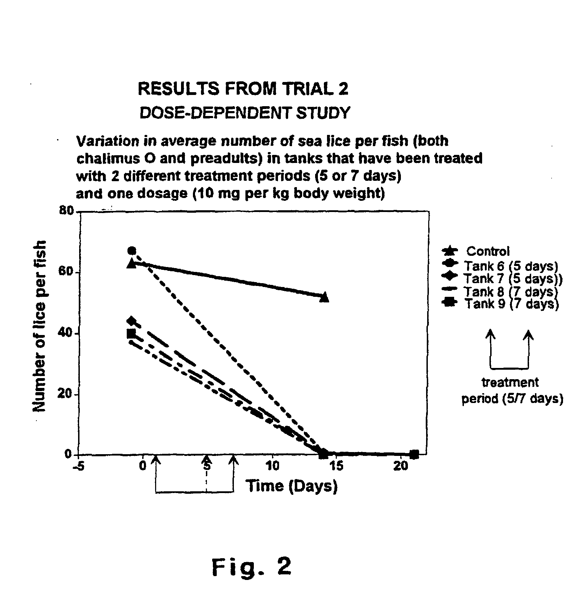

Salmon (Salmo salar) kept in tanks were artificially infected by adding a large amount of infectious larvae to the water. Seven days after the infection the fish were treated in two different periods (5 or 7 days) with 10 mg teflubenzuron per kilo body weight per day. Teflubenzuron was administered through the feed by mixing teflubenzuron and common fish feed such that teflubenzuron was spread on the surface of the feed pellets. The fish ate the feed voluntarily. The concentration of teflubenzuron in the feed was 2 g/kg feed. The water temperature was 13 °C. Figure 2 shows that the selected dose is sufficient for killing all the lice that infected the fish. The result is compared with a group of fish that was not treated.

### Trial 3

This trial was done with large salmon (Salmo salar) in a commercial fish farm. The fish were mostly infected by adult sea lice. The teflubenzuron was added through the feed by mixing teflubenzuron and ordinary fish feed such that teflubenzuron was spread on the surface of the feed pellets. The fish ate the feed voluntarily. The concentration of tefluhenzuron in the feed was 2 g/kg feed. The length of treatment was 7 days and the fish received 10 mg teflubenzuron per kilo body weight per day. The water temperature was 6-7 °C.

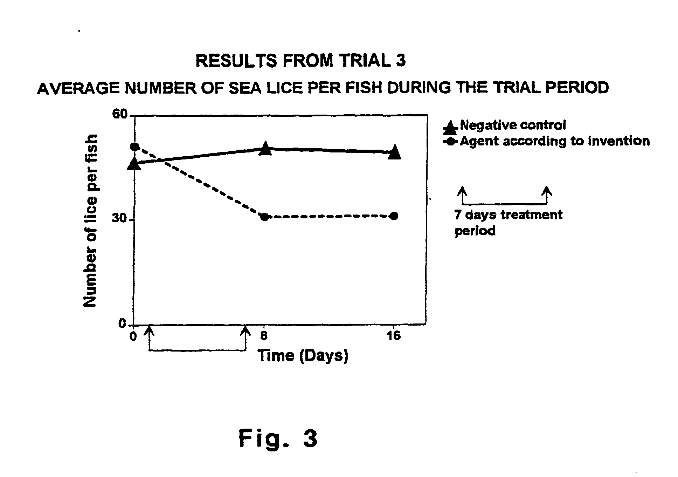

Figure 3 shows that after treatment the number of adult sea lice had been reduced to half that of the group that was not treated.

As is apparent from the trial description and the figures a significant effect has been achieved towards sea lice on salmon. The mixture is not encumbered with all the disadvantages which the familiar remedies against sea lice have. Due to the mentioned effect, a significant average increase of weight per fish will be obtained.

Teflubenzuron and possible active metabolites of teflubenzuron have therapeutical effect in fish tissues or gut and is administered orally to the fish by use of carriers like feed pellets, capsules, powder or similar containing teflubenzuron.

As mentioned earlier, teflubenzuron influences the chitin synthesis, so that chitin is not formed or synthesized chitin will not be deposited in a functional form. Chitin is a.o. present in the shell of crustaceans and in the hooks of intestinal parasites such as tapeworm.

When the fish has been given teflubenzuron, it is transferred from the stomach and gut, via the blood, to other organs of the fish and to the mucus on the gills and skin. Parasites that feed on the fish's blood, mucus, skin and/or muscles, will be exposed to the anti-parasitic mixtures. Parasites in the gut, living on the gut content, will be exposed directly to the gut contents.

Parasites that are dependent on moulting, will die during the process of moulting as a result of teflubenzuron uptake. Parasites that are dependent on synthesizing chitin, will die when the parasite is growing and is unable to create functional organs.

Teflubenzuron can be used in doses from 0.1 mg per kg body weight per day up to 1000 mg per kg body weight per day from 1 until 28 days in order to achieve the required effect. The particle size of teflubenzuron can be from 0.1 µm to 500 µm or teflubenzuron can be dissolved in a separate solution. Teflubenzuron is administered either by adding to processed fish pellets in the form of powder, granulate, dissolution, emulsions or suspensions or by adding to other raw materials in fish pellets in the form of powder, granulate, solutions, emulsions or suspensions.

Teflubenzuron can be used in combating a number of fish parasites and especially fish parasitic crustaceans, such as Copepoda with a.o. the species Caligus and Lepeophtheirus. In addition to be used for salmon, teflubenzuron can be applied for a number of related species and other fish species.

It is suggested that teflubenzuron can be added as powder, granulate, dissolution, suspension, emulsion, capsule, tablet etc. by force feeding of the fish without the use of carrier for voluntary uptake, possibly supplied through the anal opening with the same formulations. Teflubenzuron can also be administered intravenously, intraperitonally or intra musculary as a dissolution, emulsion or suspension.

## Claims

1. A composition for the oral treatment of farmed fish against chitin synthesis-dependent parasite infection consisting of teflubenzuron of the formula: 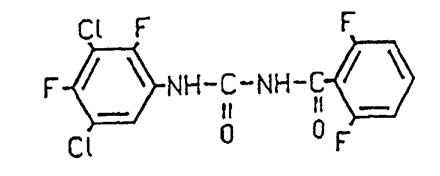 as agent for combating parasites and an edible carrier.

2. A composition as claimed in claim 1, wherein the edible carrier is fish food, preferably in the form of pellets.

3. The use of teflubenzuron of the formula: 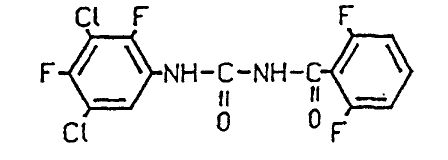 for the manufacture of a medicament for the treatment of farmed fish against chitin synthesis-dependent parasite infection.

4. A use as claimed in claim 3, wherein the medicament is for oral administration, preferably wherein the medicament is in the form of fish food, more preferably in the form of pellets.

5. A use as claimed in claim 3 or claim 4, wherein the fish are salmon.

6. A use as claimed in any of claims 3 to 5, wherein the parasites are:
(i) crustaceans,
(ii) tapeworm
(iii) sea lice,
(iv) of the family *Copopeda,* or
(v) of the species *Lepeophtheirus* or *Caligus.*

## Patentansprüche

1. Gemisch für die orale Behandlung von Zuchtfischen gegen von der Chitinsynthese abhängigen Parasiteninfektion, bestehend aus Teflubenzuron der Formel 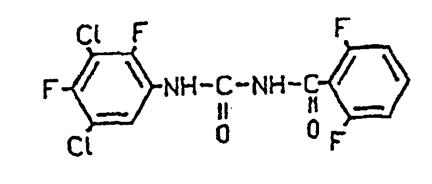 als Mittel zur Bekämpfung von Parasiten und einem genießbaren Träger.

2. Gemisch nach Anspruch 1, bei den der genießbare Träger Fischfutter, vorzugsweise in Form von Pellets, ist.

3. Verwendung von Teflubenzuron der Formel: 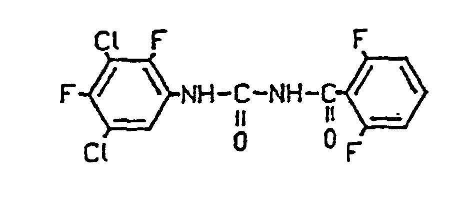 zur Herstellung eines Medikaments zur Behandlung von Zuchtfischen gegen von der Chitinsynthese abhängigen Parasiteninfektion.

4. Verwendung nach Anspruch 3, bei der das Medikament für die orale Verabreichung bestimmt ist und es vorzugsweise in Form von Fischfutter und insbesondere in Form von Pellets vorliegt.

5. Verwendung nach Anspruch 3 oder Anspruch 4, bei der der Fisch Lachs ist.

6. Verwendung nach einem der Ansprüche 3 bis 5, bei dem die Parasiten zur folgenden Gruppe gehören:
(i) Crustaceen,
(ii) Bandwurm,
(iii) Fischassel,
(iv) Vertreter der Familie der Copepoda oder
(v) Arten der Gattungen Lepeophtheirus oder Caligus.

## Revendications

1. Composition pour le traitement oral des poissons d'élevage contre l'infection par des parasites dépendant de la synthèse de la chitine constituée par le teflubenzuron de formule : 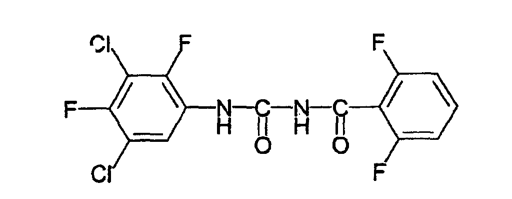 en tant qu'agent pour combattre les parasites et un porteur comestible.

2. Composition selon la revendication 1, dans laquelle le porteur comestible est de la nourriture pour poisson, de préférence sous la forme de granulés.

3. Utilisation du teflubenzuron de formule : 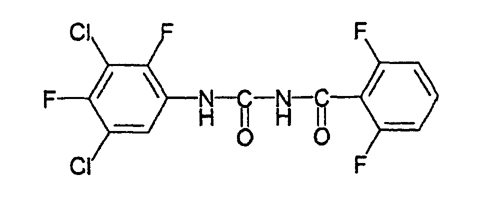 pour la fabrication d'un médicament pour le traitement des poissons d'élevage contre l'infection par des parasites dépendant de la synthèse de la chitine.

4. Utilisation selon la revendication 3, dans laquelle le médicament est prévu pour une administration par voie orale, de préférence dans laquelle le médicament est sous la forme de nourriture pour poisson, encore mieux sous la forme de granulés.

5. Utilisation selon la revendication 3 ou la revendication 4, dans laquelle le poisson est du saumon.

6. Utilisation selon l'une quelconque des revendications 3 à 5, dans laquelle les parasites sont :
(i) des crustacés,
(ii) des plathelminthes,
(iii) des poux de mer,
(iv) de la famille *Copopeda,* ou
(v) des espèces *Lepeophtheirus* ou *Caligus.*
